Europäisches Patentamt

European Patent Office

Office européen des brevets

(19)

(11) Numéro de publication: **0 253 693 B1**

(12) ## FASCICULE DE BREVET EUROPEEN

(45) Date de publication de fascicule du brevet: **06.04.94**

(51) Int. Cl.5: **C12N 15/48**, C12N 15/86, C07K 15/04, C07K 15/06, G01N 33/569, A61K 39/21, A61K 39/285

(21) Numéro de dépôt: **87401338.6**

(22) Date de dépôt: **15.06.87**

(54) **Vaccin, contenant la protéine F du virus du SIDA.**

(30) Priorité: **16.06.86 FR 8608698**

(43) Date de publication de la demande:
**20.01.88 Bulletin 88/03**

(45) Mention de la délivrance du brevet:
**06.04.94 Bulletin 94/14**

(84) Etats contractants désignés:
**AT BE CH DE ES FR GB GR IT LI LU NL SE**

(56) Documents cités:

**PROCEEDINGS OF THE NATIONAL ACADEMY OF SCIENCES USA, vol. 83, avril 1986, pages 2209-2213, US; S.K. ARYA et al.: "Three novel genes of human T-lymphotropic virus type III: immune reactivity of their products with sera from acquired immune deficiency syndrome patients"**

**NATURE, vol. 312, no. 5990, 8 novembre 1984, pages 163-166, Reading, Berks, GB; M.P. KIENY et al.: "Expression of rabies virus glycoprotein from a recombinant vaccinia virus"**

(73) Titulaire: **TRANSGENE S.A.**
**11, rue de Molsheim**
**F-67000 Strasbourg(FR)**

Titulaire: **INSTITUT PASTEUR**
**25/28, rue du Docteur Roux**
**F-75015 Paris(FR)**

(72) Inventeur: **Kieny, Marie-Paule**
**1, rue de Gascogne**
**F-67100 Strasbourg(FR)**
Inventeur: **Guy, Bruno**
**18, rue Sainte-Madeleine**
**F-67000 Strasbourg(FR)**
Inventeur: **Lecocq, Jean-Pierre**
**6, rue du Champs du Feu**
**F-67116 Reichstett(FR)**
Inventeur: **Montagnier, Luc**
**21, rue de Malabry**
**F-92350 Le Plessis-Robinson(FR)**

NATURE, vol. 320, 10 avril 1986, pages 537-540, Reading, Berks, GB; SHIU-LOK HU et al.: "Expression of AIDS virus envelope gene in recombinant vaccinia viruses"

NATURE, vol. 320, 10 avril 1986, pages 535-537, Reading, Berks, GB; SEKHAR CHAK-RABARTI et al.: "Expression of the HTLV-III envelope gene by a recombinant vaccinia virus"

Review: Vaccinia virus expression vectors, p. 2075

(74) Mandataire: **Warcoin, Jacques et al**
**Cabinet Régimbeau**
**26, avenue Kléber**
**F-75116 Paris (FR)**

**Description**

La présente invention concerne plus particulièrement un vaccin destiné à la prévention du SIDA.

Le syndrome d'immunodéficience acquise (SIDA) est une affection virale qui présente maintenant une importance majeure en Amérique du Nord, en Europe et en Afrique centrale.

Les estimations récentes suggèrent qu'environ 1 million d'américains peuvent avoir été exposés au virus du SIDA. Les individus affectés présentent une immunodépression sévère et la maladie est, en général, fatale.

La transmission de la maladie s'effectue le plus souvent par contact sexuel, bien que les personnes utilisant des stupéfiants par voie intraveineuse représentent également un groupe à haut risque ; d'autre part, un grand nombre d'invidus ont été infectés par ce virus après avoir reçu du sang ou des produits sanguins contaminés.

L'agent causal de cette affection est un rétrovirus. De nombreuses affections animales ont été attribuées aux rétrovirus, mais c'est seulement récemment que des rétrovirus affectant des hommes ont pu être décrits.

Alors que des rétrovirus des cellules T humaines (HTLV : human T leukemia virus) de types I et II ont été impliqués comme agent causal de certaines leucémies des cellules T chez les adultes, le rétrovirus associés à des lymphadénopathies (virus LAV), qui est également appelé virus HTLV III ou A.I.D.S.-related virus (ARV), est maintenant couramment accepté comme l'agent responsable du SIDA.

Le génome de plusieurs isolats du rétrovirus LAV ou HTLV III a été caractérisé de façon très complète (Wain-Hobson et al., 1985 ; Ratner et al., 1985 ; Muesing et al., 1985 ; Sanchez-pescador et al., 1985) et des informations sur la séquence génomique indiquent une relation étroite avec le groupe des lentivirus. Les lentivirus, dont le prototype est le virus ovin Visna, sont les agents de maladies à progression très lente et qui présentent typiquement une période d'incubation prolongée. Le LAV et le virus Visna partagent de nombreuses similarités, en particulier dans leur tropisme pour le tissu neural.

En plus des trois parties de génome retrouvées chez tous les rétrovirus et désignées gag, pol et env, le virus LAV comporte au moins trois autres gènes dénommés Q ou Ser, TAT et F ou 3'orf (Wain-Obson et al., 1985 ; Arya et al., 1986). Le produit du gène F a été caractérisé par réaction avec des serums de malades atteints de SIDA et l'identité de la protéine confirmée par un séquençage direct des acides aminés (Allan et al., 1985).

Selon les auteurs, la protéine F est reconnue par 30 à 90 % des sérums de malades. Cette protéine migre sur un gel d'acrylamide SDS selon un poids moléculaire apparent compris entre 26 et 28 kD. Certains auteurs ont mis en évidence un produit du gène F compris entre 24 et 25 kD, évoquant la possibilité d'une initiation de la traduction au deuxième ATG.

La protéine F n'est pas glycosylée, bien que deux sites potentiels de N-glycosylation soient présents. Enfin, il a été démontré que la protéine F est acylée par l'acide myristique. Il existe sur la protéine F un tetrapeptide (Arg - Phe - Asp - Ser), retrouvé sur certaines protéines intervenant dans l'adhésion cellulaire, et également sur une chaîne de l'antigène HLA de classe II.

Auffray (Auffray, 1986) a évoqué la possibilité pour la protéine F de jouer un rôle dans la reconnaissance et l'attachement de la protéine env et du virus LAV aux lymphocytes T4, contribuant ainsi à la dissémination du virus. De plus, la variabilité élevée (2 à 17 %) de la protéine F d'une souche à l'autre, variabilité comparable à celle de la glycoprotéine d'enveloppe, laisse à penser que la protéine F joue effectivement un rôle dans l'infection (Ratner et al., 1985).

La présente invention propose d'utiliser comme vecteur d'expression de la protéine F un vecteur viral permettant l'expression de la protéine dans un environnement qui permettra sa restructuration post-traductionnelle.

C'est pourquoi la présente invention concerne un vecteur viral, caractérisé en ce qu'il comporte le gène F du virus responsable du SIDA.

Parmi les vecteurs viraux utilisables, il faut citer plus particulièrement les poxvirus et, notamment, le virus de la vaccine (VV).

Le virus de la vaccine est un virus à ADN double brin qui a été utilisé très largement dans le monde entier pour contrôler et éradiquer la variole. Des développements techniques récents ont permis le développement de ce virus comme vecteur de clonage et des virus recombinants vivants ont permis d'exprimer des antigènes étrangers et même d'obtenir des immunisations contre différentes maladies virales ou parasitaires.

Ainsi, plusieurs groupes ont récemment mis en évidence l'utilisation de recombinants de ce type pour exprimer l'antigène de l'influenza, de l'hépatite B et la glycoprotéine de la rage pour immuniser contre ces maladies (Smith et al., 1983 ; Panicali et al., 1983 ; Kiney et al., 1984).

L'expression d'une séquence codant pour une protéine étrangère par le virus de la vaccine (VV) implique nécessairement deux étapes :

1°) la séquence codante doit être alignée avec un promoteur de VV et être insérée dans un segment non essentiel de l'ADN de VV, cloné dans un plasmide bactérien approprié ;

2°) les séquences d'ADN de VV situées de part et d'autre de la séquence codante doivent permettre des recombinaisons homologues in vivo entre le plasmide et le génome viral ; une double recombinaison réciproque conduit à un transfert de l'insert d'ADN du plasmide dans le génome viral dans lequel il est propagé et exprimé (Panicali et Paoletti, 1982 ; Mackett et al. 1982 ; Smith et al., 1983 ; Panicali et al., 1983).

Bien entendu, l'utilisation de ce type de vecteur implique souvent une délétion partielle du génome du virus vecteur.

La présente invention concerne plus particulièrement un vecteur viral caractérisé en ce qu'il comporte au moins :

- une partie du génome d'un virus vecteur,
- un gène codant pour la protéine F du virus responsable du SIDA,
- ainsi que les éléments assurant l'expression de cette protéine dans les cellules et une séquence signal et/ou une séquence transmembranaire provenant d'un virus hétérologue

L'invention concerne également les ADN recombinants correspondant auxdits vecteurs viraux.

Par "virus responsable du SIDA", on entend notamment désigner le virus LAV, le virus HTLV III ou ARV, de même que d'éventuels mutants ponctuels ou des délétions partielles de ces virus, ainsi que les virus apparentés.

Les vecteurs viraux, dans la partie correspondant au génome du virus vecteur (distinct du virus responsable du SIDA), peuvent être constitués à partir du génome d'un virus d'origine quelconque. Toutefois, on préférera utiliser une partie du génome d'un poxvirus et plus particulièrement une partie du génome de la vaccine.

Les conditions nécessaires pour l'expression d'une protéine hétérologue dans le virus de la vaccine ont été rappelées précédemment.

De façon générale, le gène en cause, par exemple le gène F, devra, pour pouvoir être exprimé, être sous la dépendance d'un promoteur d'un gène de la vaccine, ce promoteur sera en général le promoteur de la protéine 7,5K de la vaccine. En outre, la séquence codante devra être clonée dans un gène non essentiel de la vaccine qui pourra éventuellement servir de gène marqueur. Dans la plupart des cas, il s'agira du gène TK.

La protéine F native ne possède pas de signal d'excrétion et n'est pas glycosylée. On propose ici des modifications de ce gène pour améliorer l'immunogénicité du produit d'expression.

On propose de modifier le gène F de façon à assurer son excrétion hors de la cellule, et, d'autre part, pour une troisième construction, à assurer l'ancrage de la protéine dans la membrane.

Pour ce faire, le gène pourra être modifié au niveau de sa partie codant pour l'extrémité $NH_2$ terminale de la protéine en ajoutant une séquence signal qui provient d'un virus hétérologue, par exemple la séquence signal de la glycoprotéine du virus rabique. Ceci permettra l'excrétion de la protéine hors de la cellule.

Il est bien entendu que l'on peut utiliser une séquence signal provenant d'autres protéines virales.

Enfin, il peut être intéressant pour l'immunogénicité de la protéine que celle-ci soit ancrée dans la membrane cellulaire et ainsi correctement présentée au système immunitaire de l'hôte. C'est pourquoi il est proposé, à partir de la précédente construction (F + signal rage), d'ajouter au niveau de la partie codant pour l'extrémité COOH terminale de la protéine F une séquence codant pour la portion transmembranaire provenant d'un virus hétérologue, celle de la glycoprotéine du virus rabique également.

La présente invention concerne tout d'abord l'utilisation de vecteurs viraux pour l'obtention de la protéine codée par le gène F du virus LAV dans des cultures cellulaires.

Il s'agit donc dans un premier temps de cellules de mammifères qui ont été infectées par un vecteur viral selon l'invention ou bien qui peuvent contenir l'ADN recombinant correspondant ; parmi ces cellules, il faut citer plus particulièrement les cellules diploïdes humaines, des cultures primaires ainsi que les cellules Vero. Bien entendu, il est possible de prévoir d'autres types de cellules comme cela ressortira d'ailleurs des exemples ci-après.

Les protéines ainsi obtenues peuvent être utilisées après purification pour la réalisation de vaccins.

Il est également possible de prévoir l'utilisation directe des vecteurs viraux selon l'invention afin d'effectuer une vaccination, la protéine F étant alors produite in situ et in vivo.

La présente invention concerne également les anticorps dressés contre les protéines F précédentes obtenus par infection d'un organisme vivant avec un vecteur viral tel que décrit précédemment et récupération des anticorps induits après un temps déterminé.

Enfin, la présente invention concerne la détection des anticorps dressés contre les protéines F précédentes ainsi que le procédé de diagnostic correspondant permettant, à partir de la détection de ces anticorps dans le prélèvement biologique d'un patient, de détecter in vitro le SIDA chez ce patient.

Les techniques mises en oeuvre pour l'obtention de la protéine F, les cultures cellulaires et les techniques de vaccination sont identiques à celles qui sont pratiquées actuellement avec les vaccins connus et ne seront pas décrites en détail.

La présente invention sera mieux comprise à la lecture des méthodes et exemples suivants.

Les figures suivantes illustrent les exemples :

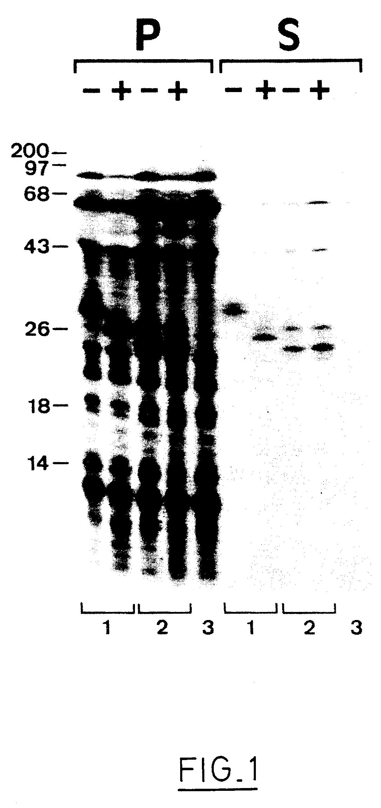

. la figure 1 représente l'action de la tunicamycine sur les protéines synthétisées par les recombinants VV.TG.FLAV.II47 et VV.TG.FLAV.II45 et immunoprécipitées grâce à un sérum anti-LAV ; dans cette figure, les poids moléculaires sont donnés en kiloDaltons, et on représente par :

    P :     le culot cellulaire

    S :     le surnageant

    - :     les produits obtenus sans traitement

    + :     les produits obtenus après traitement à la tunicamycine

    1 :     VV.TG.FLAV.II45

    2 :     VV.TG.FLAV.II47

    3 :     virus de la vaccine, type sauvage ;

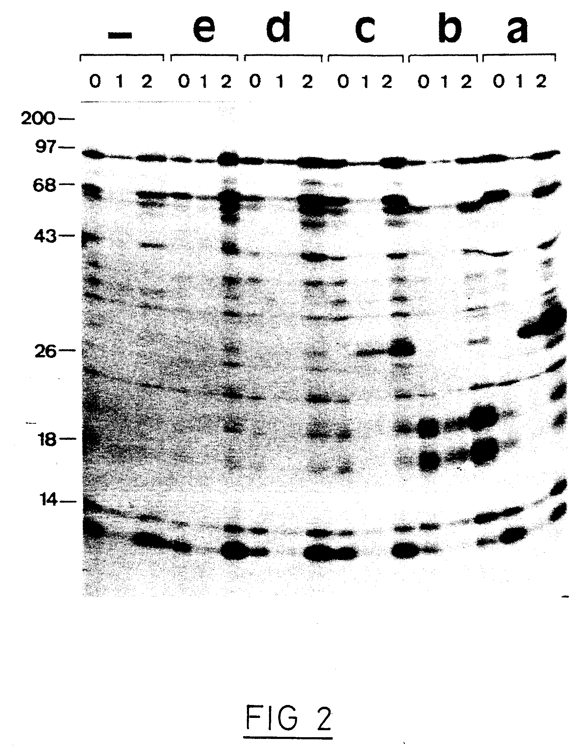

. la figure 2 représente le criblage de sérums anti-LAV pour leur capacité à reconnaître la protéine F synthétisée par le virus recombinant VV.TG.FLAV.II45 ; les poids moléculaires sont exprimés en kilodaltons, et on représente par :

    . 0 : virus de la vaccine, type sauvage

    . 1 : VV.TG.FLAV.II45, clone C

    . 2 : VV.TG.FLAV.II45, clone D

    . - : sérum témoin négatif

    . a, b, c, d, e : sérums de malades atteints de SIDA ;

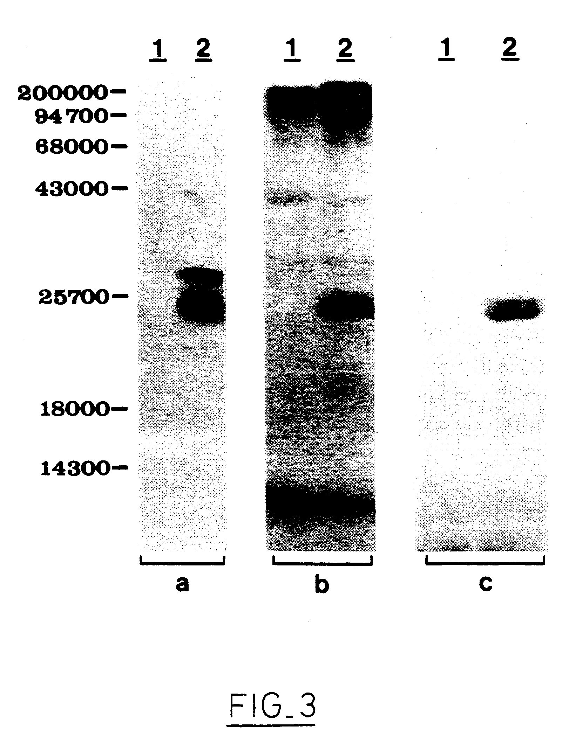

. la figure 3 représente l'immunoprécipitation des protéines synthétisées par le recombinant VV.TG.II47 ; sur cette figure on désigne par :

    . 1 : le virus vaccinal sauvage

    . 2 : VV.TG.FLAV.II47

    . a : le marquage des cellules BHK2I à la méthionine $^{35}$S

    . b : le marquage des cellules BHK2I au $^{32}$PO$_4$

    . c : le marquage des cellules BHK2I à l'acide myristique $^{3}$H

les poids moléculaires sont en daltons ;

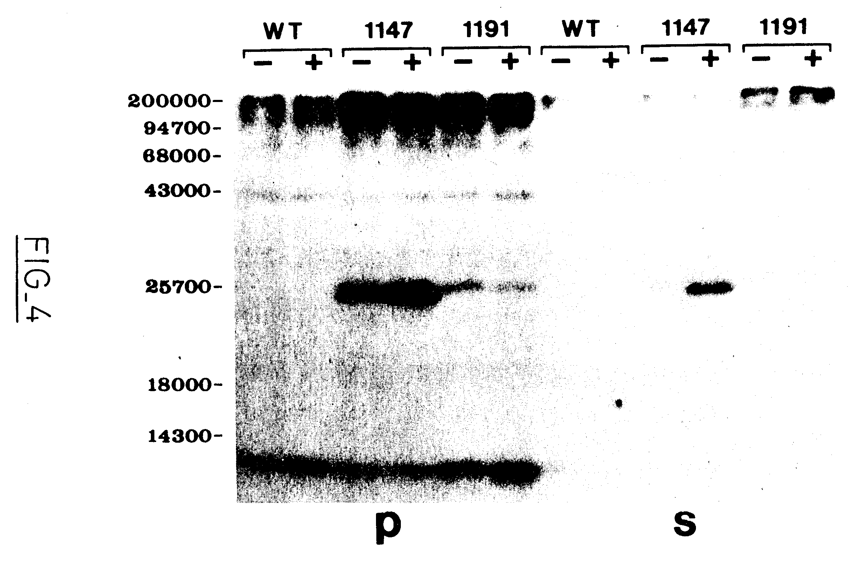

. la figure 4 représente l'immunoprécipitation d'extraits de cellules BHK2I infectées par les recombinants vaccinaux après marquage au $^{32}$PO$_4$ ; sur cette figure on désigne par :

    . - : l'immunoprécipitation sans addition de TPA

    . + : l'immunoprécipitation avec addition de TPA

    . P : le culot cellulaire

    . S : le surnageant de culture

les poids moléculaires sont en daltons ;

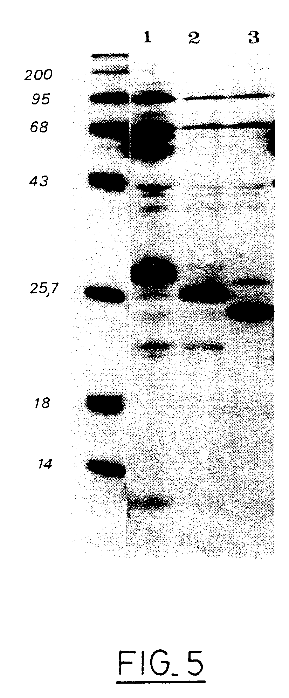

. la figure 5 représente l'immunoprécipitation des protéines synthétisées par le virus recombinant VV.TG.FLAV.II65 et marquées à la méthionine $^{35}$S ; sur cette figure on désigne par :

    . 1 : les cellules infectées par VV.TG.FLAV.II45

    . 2 : les cellules infectées par VV.TG.FLAV.II65

    . 3 : les cellules infectées par VV.TG.FLAV.II47

les poids moléculaires sont en kilodaltons ;

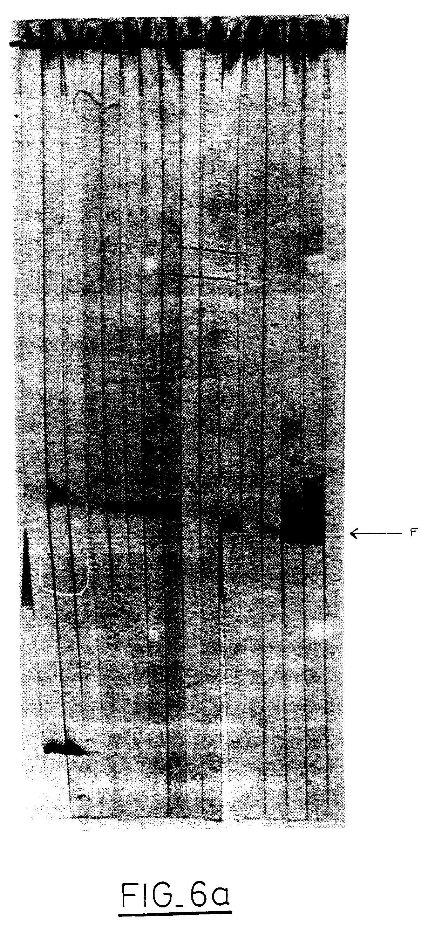

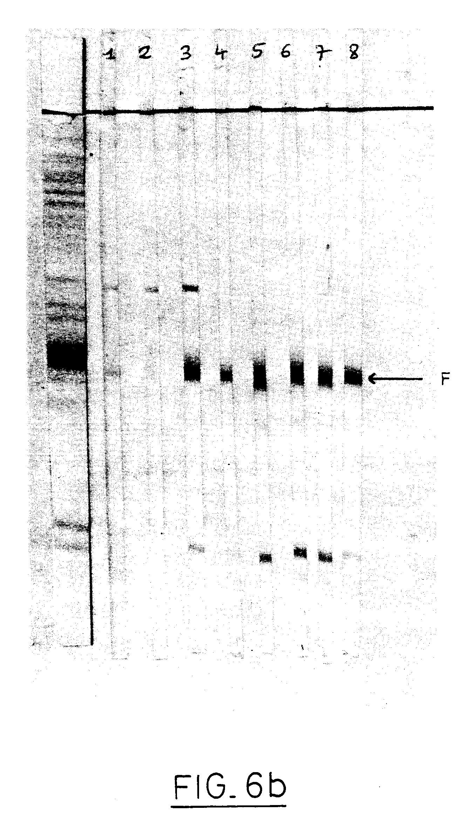

. les figures 6a et 6b représentent la réaction des anticorps de souris vaccinées avec les virus recombinants vis-à-vis de la protéine F produite dans E. coli et transférée sur nitrocellulose ;

sur la figure 6a :

    . les bandes 1 et 3 représentent le virus vaccine sauvage

    . les bandes 2, 4, 5, 8, 9 représentent le virus VV.TG.FLAV.II45

    . les bandes 6, 7, 8, 11, 12 représentent le virus VV.TG.FLAV.II47

    . les bandes 13, 14, 15 représentent le virus VV.TG.FLAV.II46 ;

sur la figure 6b :

    . les bandes 1, 2 représentent le virus vaccine sauvage

    . la bande 3 représente le virus VV.TG.FLAV.II46

. la bande 4 représente le virus VV.TG.FLAV.II47
. les bandes 5, 6, 7, 8 représentent le virus VV.TG.FLAV.II65 .

**METHODES**Clonage : Maniatis et al., 1982.
Enzymes : Utilisées selon les prescriptions du fournisseur.
Mutagénèse localisée : Méthode dérivée de Zoller et Smith, 1983.
Transfert dans la vaccine : Kieny et al., 1984. Seule différence : les cellules humaines I43B remplacent les cellules LMTK⁻.

Préparation du stock de virus

Les cellules primaires de poulet "germ free" sont infectées à 0,01 pfu/cellule pendant 4 jours à une température de 37°C (milieu MEM + 5 % NCS).

Purification du virus

On effectue une centrifugation du stock de virus ci-dessus pendant 15 minutes à 2 500 tours (Rotor GSA Sorvall). Le surnageant est mis de côté. On reprend le culot dans un tampon RSB (Tris HCl 10 mM pH 7,4, KCl 10 mM, MgCl$_2$ 1 mM) pendant 15 minutes à 4°C. On effectue un broyage au potter, puis une centrifugation pendant 15 minutes à 2 500 tours. Le surnageant est ajouté au précédent puis on effectue un deuxième broyage de la même façon.

Tous les surnageants sont déposés sur 10 ml de coussin de saccharose 36 % (p/v) (Tris 10 mM pH 8). On effectue une centrifugation pendant 2 heures à 14 000 tours (Rotor SW28, Beckman).

Le culot est repris, dissocié et remis sur un deuxième coussin identique. Le deuxième culot est repris dans 5 ml PBS et chargé sur un gradient 20-40 % de saccharose (Tris 10 mM pH 8) (même rotor). On effectue une centrifugation pendant 45 minutes à 12 000 tours.

On récupère la bande de virus -. Elle est culottée par centrifugation pendant 1 heure à 20 000 tours. Le culot est repris dans du Tris 10 mM pH 8.

Immunoprécipitations

On effectue une infection de cellules BHK2l (boîtes de 3 cm de diamètre, 10$^6$ cellules/boîte, cultivées en G-MEM + 10 % FCS) à 0,2 pfu/cellule pendant 18 heures. Le milieu est décanté et remplacé par 1 ml de milieu sans méthionine et 10 $\mu$l de méthionine $^{35}$S (5 mCi/300 $\mu$l) (Amersham) par boîte.

On ajoute un excès de méthionine non radioactive après 2 heures.

A la fin du marquage, on effectue un grattage des cellules infectées, une centrifugation pendant 1 minute dans une centrifugeuse Eppendorf, une séparation des fractions surnageant et culot, un lavage du culot une fois en tampon PBS, puis une immunoprécipitation et un gel (Selon Lathe et al., 1980).

Immunoprécipitation des protéines synthétisées en présence de tunicamycine

Après infection de cellules BHK2l comme décrit précédemment, on ajoute après 18 heures 1,5 $\mu$g de tunicamycine par ml de milieu pendant 1 heure.

Le milieu est ensuite décanté et remplacé par 1 ml de milieu MEM sans méthionine contenant 1,5 $\mu$g de tunicamycine et 10 $\mu$l de méthionine $^{35}$S (Amersham). La suite de la manipulation s'effectue comme précédemment.

Traitement endo-F

Après immunoprécipitation des protéines marquées par un sérum de malade atteint du SIDA, la fraction protéine-A sepharose est reprise dans :
0,2 M phosphate de Na, pH 6,1
0,05 % SDS
0,1 % Nonidet P40
0,1 % Beta-mercaptoéthanol
0,1 % EDTA pH 8
et bouillie pendant 5 minutes pour dénaturer les protéines.

On effectue une incubation pendant 20 heures à 37°C, avec 4 unités d'endo-F par ml, puis une précipitation pendant 2 minutes dans la glace avec 1/5 de volume de TCA 100 %. On lave le culot 3 fois à l'acétone 80 %, on ajoute le tampon d'échantillons et on charge sur gel SDS.

Dosage des anticorps vaccine par test ELISA

Des plaques 96 trous (NUNC) à fond plat sont incubées pendant 18 heures à 37°C avec $10^6$ pfu de virus de la vaccine type sauvage en tampon carbonate. Les plaques sont ensuite saturées avec de la gélatine 0,01 %. Les sérums de souris sont alors adsorbés sur les plaques et on effectue le reste du protocole comme pour un test ELISA classique.

Lectures à 492 nM.

**EXEMPLE 1**

Construction des plasmides hybrides

Les tailles combinées des différents éléments nécessaires pour le transfert de la séquence codant pour le gène env dans le génome de VV et son expression subséquente sont de l'ordre de plusieurs kb. Il a donc été jugé nécessaire de minimiser la taille du plasmide de réplication dans E. coli utilisé pour le travail de construction de façon à faciliter les manipulations nécessaires.

Le fragment HindIII (Hin-J) du génome de VV contient le gène complet de la thymidine kinase (TK) qui a déjà été utilisé précédemment pour permettre l'échange et la recombinaison de l'ADN inséré dans le génome de VV (Macket et al., 1982). Il est important de noter que le transfert d'un insert dans le gène TK du génome de VV crée un virus TK déficient qui peut être sélectionné. Il a tout d'abord été nécessaire de produire un plasmide de petite taille portant un site unique HindIII utilisable pour l'intégration du fragment Hin-J VV. En outre, il était nécessaire d'éliminer les séquences de restriction non nécessaires du plasmide de façon à permettre les manipulations suivantes.

La construction a été amorcée à partir du plasmide pML2 (Lusky et Botchan, 1981) qui est un vecteur dérivé du plasmide pBR322 par délétion spontanée dans lequel le segment entre les nucléotides 1089 et 249I a été perdu. D'abord la séquence de PstI a été éliminée par insertion du fragment AhaIII-AhaIII de pUC8 (Vieira et Messing, 1982) entre deux sites AhaIII de pML2 en éliminant 19 paires de bases. On a utilisé la méthode du "linker-tailing" (Lathe et al., 1984) pour insérer un linker HindIII entre les sites NruI et EcoRI traite par SI de ce plasmide, en éliminant le site BamHI.

Ceci conduit à un plasmide de 2049 paires de bases portant le gène betalactamase fonctionnel (conférant la résistance à l'ampicilline) et comportant en outre une origine de réplication active dans E. coli et un site de restriction unique HindIII.

Cette construction a été appelée pTGIH.

Le fragment Hin-J de l'ADN de VV portant le gène TK a préalablement été cloné dans un vecteur provenant de pBR327 (Drillien et Spehner, 1983). Ce fragment de 4,6 kb a été recloné dans le site HindIII de pTGIH. Un clone a été sélectionné dans lequel le gène TK est situé distalement par rapport au gène codant pour la résistance à l'ampicilline.

Cette construction pTGIH-TK a été utilisée comme vecteur dans l'expérience suivante.

L'étape suivante a été d'isoler un promoteur de VV utilisable pour commander l'expression de la séquence codant pour le gène à exprimer. Le promoteur d'une gène précoce codant pour une protéine de 7 500 daltons (7,5K) a déjà été utilisé avec succès dans un but identique (Smith et al., 1983) et on a donc procédé à l'isolement de ce segment.

Le gène 7,5K est situé sur l'un des plus petits fragments SalI (fragment Sal-S) du génome de VV type WR (Venkatasan et al., 1981). Comme les petits fragments sont clonés de façon préférentielle, une grande proportion des clones obtenus par clonage direct de l'ADN de VV type WR coupé par SalI dans le plasmide pBR322 porte le fragment Sal-S. Ce fragment est transféré sur le bactériophage vecteur Ml3mp70I (Kieny et al., 1983) par digestion SalI et religation, en conduisant ainsi au phage Ml3TGSal-S.

Dans ce clone, un site ScaI se trouve immédiatement à proximité de l'ATG d'initiation du gène 7,5K. En aval du gène 7,5K se trouvent situés des sites uniques BamHI et EcoRI provenant du vecteur. Les sites BamHI et SCaI sont fusionnés par l'intermédiaire d'un linker BglII 5'-CAGATCTG-3' après avoir complété les extrémités générées par digestion BamHI avec le fragment Klenow de la polymérase de E. coli. Ce procédé élimine le site ScaI mais reconstitue le site BamHI et déplace le site unique EcoRI en aval. En même temps, le site SalI (AccI) en aval est éliminé, le site SalI en amont devient donc unique.

Cette construction est appelée Ml3TG 7,5K.

A l'intérieur du fragment Hind-J de l'ADN de VV se trouvent situés des sites Call et EcoRI qui sont séparés par environ 30 paires de bases (Weir et Moss, 1983). Le fragment promoteur de 7,5K présent dans MI3TG7,5K est excisé par AccI et EcoRI et cloné entre les sites ClaI et EcoRI de pTGIH-TK pour générer pTGIH-TK-P7,5K.

Cette construction conduit au transfert des sites BamHI et EcoRI uniques du vecteur MI3 immédiatement en aval de la séquence du promoteur 7,5K. Ces sites uniques BamHI et EcoRI sont utilisés dans la construction suivante.

Le segment polylinker du bactériophage MI3TGI3I (Kieny et al., 1983) est excisé par EcoRI et BgIII et inséré entre les sites EcoRI et BamHI du plasmide pTGIH-TK-P7,5K, générant pTGI86-poly. Dans cette construction, 5 sites de restriction uniques sont disponibles pour le clonage d'un gène étranger sous le contrôle de P7,5K (PstI, BamHI, SstI, SmaI, EcoRI).

## EXEMPLE 2

Construction du plasmide portant le séquence F

Tout d'abord, le gène env du LAV entouré par les séquences signal et transmembranaire de la glycoprotéine du virus rabique a été inséré dans un plasmide (pTGII34) précédemment décrit. Le fragment PstI-PstI du plasmide pTGII34 est cloné dans le vecteur MI3mp70I dans une orientation adéquate. Le bactériophage résultant est MI3TGI69 :

(SR = signal rage

(TMR = transmembranaire rage

Le fragment génomique codant pour la protéine F est obtenu par une digestion BamHI-HindIII du plasmide pJI9-6, segment proviral cloné et décrit précédemment.

Le fragment BamHI-HindIII de pJI9-6 est cloné dans le vecteur MI3TGI69 ouvert aux sites KpnI et HindIII. Le site BamHI est relié au site KpnI par un linker oligonucléotide simple brin de séquence :

5'-GATCGTAC-3'

Le bactériophage résultant est appelé MI3TGI70 :

8

Afin de pouvoir exciser uniquement la séquence codante de la protéine F, un site BamHI est généré en amont de l'ATG d'initiation de la traduction, par mutagénèse dirigée par oligonucléotide.

```
      `ATG      F
  //⌐————┬————————————→————————————//        M13TG17O (simple brin)
   ∟————┴
      ⌄
```

La séquence de l'oligonucléotide est :

<div align="center">

5' GAAAGGATCCTGCTATAAG 3'
BamHI

</div>

Après réparation et criblage des plages par l'oligonucléotide marqué au $^{32}$P, le phage MI3TGI73 est isolé. Il ne diffère du phage MI3TGI70 que par le site BamHI situé en amont de l'ATG d'initiation.

```
        ATG      F        TGA
  //┼————┼——————→————————┼——————┼————//        (M13TG173)
    BamHI                SstI
```

L'introduction de ce site permet de cloner le fragment BamHI-SstI qui contient le gène F dans le plasmide de transfert pTGI86-poly ouvert aux sites BamHI et SstI et dont la construction a été décrite précédemment. Comme cela a été indiqué, l'expression d'une protéine hétérologue dans le virus de la vaccine nécessite que la séquence codante soit alignée avec une séquence du promoteur de la vaccine et soit insérée dans un segment non essentiel de l'ADN de la vaccine. Cet ADN situé de part et d'autre permet la recombinaison avec le génome de la vaccine in vivo par une double recombinaison réciproque qui transfère la séquence codante et le promoteur accompagnant dans le génome de la vaccine. Le plasmide résultant est le plasmide pTGII47.

Le transfert de la séquence codante du gène F et du promoteur accompagnant dans le génome de la vaccine est effectué comme suit :

**EXEMPLE 3**

Clonage dans le virus de la vaccine pour générer VV.TG.FLAV.II47

La stratégie décrite par Smith et al., 1983 repose sur l'échange in vivo entre un plasmide portant un insert dans le gène VV TK et le génome viral de type sauvage de façon à inactiver le gène TK porté par le virus. Les virus TK⁻ peuvent être sélectionnés par étalement sur une lignée cellulaire (TK négative) en présence de 5-bromodéoxyuridine (5BUDR) (Mackett et al., 1982). La thymidine kinase phosphoryle le 5BUDR en 5'-monophosphate, qui est ensuite converti en triphosphate. Ce composé est un analogue de dTTP et son incorporation dans l'ADN bloque le développement correct du virus. Un virus TK- peut néanmoins répliquer son ADN normalement et il conduit à des plages virales visibles dans une lignée cellulaire également TK⁻.

Le virus de la vaccine se propage dans le cytoplasme des cellules infectées plutôt que dans leur noyau. C'est pourquoi il n'est pas possible de tirer avantage de la machinerie de réplication et de transcription de l'ADN de l'hôte et il est nécessaire que le virion possède les composants pour l'expression de son génome. L'ADN de VV purifié est non infectieux.

Afin de générer les recombinants, il est nécessaire d'effectuer simultanément l'infection cellulaire avec du virion VV et une transfection avec le segment d'ADN cloné qui présente de l'intérêt. Toutefois, la génération des recombinants est limitée à la petite proportion des cellules qui sont compétentes pour la transfection par l'ADN. C'est pour cette raison qu'il a été nécessaire de mettre en oeuvre une stratégie de "congruence" indirecte pour réduire le bruit de fond des virus parentaux non-recombinants. Ceci a été

<div align="center">9</div>

effectué en utilisant comme virus infectieux vivant un mutant thermosensible (ts) de la vaccine qui n'est pas capable de se propager à une température non permissive de 39,5°C (Drillien et Spehner, 1983). Lorsque les cellules sont infectées par un mutant ts dans des conditions non permissives et transfectées avec l'ADN d'un virus de type sauvage, la multiplication virale interviendra seulement dans les cellules qui sont compétentes pour la transfection et dans lesquelles une recombinaison entre l'ADN viral sauvage et le génome du virus ts aura eu lieu ; aucun virus ne se multipliera dans les autres cellules, en dépit du fait qu'elles ont été infectées. Si un plasmide recombinant contenant un fragment de l'ADN de vaccine tel que pTGII47 est inclus dans le mélange de transfection, à la concentration appropriée, avec l'ADN du type sauvage, il est également possible d'obtenir qu'il participe à la recombinaison homologue avec l'ADN de la vaccine dans les cellules compétentes.

Des monocouches de cellules primaires de fibroblastes d'embryons de poulets (CEF) sont infectées à 33°C avec VV-Copenhague ts7 (0,1 pfu/cellule) et transfectées avec un coprécipité au phosphate de calcium de l'ADN du virus de type sauvage VV-copenhague (50 ng/$10^6$ cellules) et le plasmide recombinant (50 ng/$10^6$ cellules).

Après incubation pendant 2 heures à une température qui ne permet pas le développement du virus ts (39,5°C), les cellules sont incubées de nouveau pendant 48 heures à 39,5°C. Des dilutions de virus ts$^+$ sont utilisées pour réinfecter une monocouche de cellules humaines I43B-TK$^-$ à 37°C qui sont ensuite incubées en présence de 5BUDR (150 $\mu$g/ml). Différentes plaques de virus TK$^-$ sont obtenues à partir de ces cellules qui ont reçu le plasmide recombinant, tandis que les cultures contrôles sans plasmide ne montrent pas de plaques visibles. Les virus TK$^-$ sont ensuite sous-clonés par une deuxième sélection en présence de 5BUDR.

Une double recombinaison réciproque correcte entre le plasmide hybride pTGII47 et le génome de VV aboutit à l'échange du gène TK portant l'insert avec le gène TK du virus, les recombinants devenant ainsi TK$^-$.

Les ADN purifiés à partir des différents virus recombinants TK$^-$ sont digérés par HindIII et soumis à une électrophorèse sur gel d'agarose. Les fragments d'ADN sont transférés sur un filtre de nitrocellulose selon la technique décrite par Southern (1975). Le filtre est ensuite hybridé avec le plasmide pTGII47 nick-translaté au $^{32}$P. Après lavage du filtre, ce dernier est fluorographié et des bandes de 3,85, 2,9, 0,8 kb sont visibles sur l'autoradiographie quand le virus vaccine a incorporé le gène F du LAV. L'un de ces recombinants VV.TG.FLAV.II47 a été sélectionné pour les études suivantes.

### EXEMPLE 4

Protéine F synthétisée à partir d'un virus recombinant vaccine-LAV

Pour mettre en évidence l'expression du gène F du LAV à partir du virus vaccine hybride, on infecte des cellules de rongeur, BHK2I, qui sont cultivées dans un milieu G-MEM + 10 % de sérum de veau foetal avec ledit recombinant VV.TG.FLAV.II47.

Une monocouche semi-confluente fraîche ($10^6$ cellules) est infectée avec 0,2 pfu/cellule et incubée pendant 18 heures.

Le milieu est ensuite éliminé et on ajoute un milieu à faible teneur en méthionine (1 ml pour $10^6$ cellules) supplémenté avec 10 $\mu$l/ml de méthionine $^{35}$S (5 mCi/300 $\mu$l). Les cellules sont incubées à 37°C et les protéines marquées sont collectées par centrifugation. Après séparation en culot et surnageant, les protéines sont incubées avec un sérum appartenant à des patients atteints de SIDA. Les protéines réagissant avec le sérum sont récupérées par adsorption sur une résine protéine A-sépharose et étalées par électrophorèse sur un gel de polyacrylamide SDS et auto-radiographiées selon une technique décrite par Lathe et al., 1980. Les autoradiographies montrent que certains sérums des patients atteints du SIDA lient spécifiquement deux protéines des extraits cellulaires infectés. Les poids moléculaires apparents de 25,5 et 27 kd suggèrent l'équivalence avec la protéine F identifiée par des sérums de malades atteints du SIDA, dans une préparation de protéine F authentique et dans des extraits de cellules infectées par le virus LAV.

La séquence codant pour F conduit à un produit de traduction primaire d'environ 23 kd alors que la protéine F obtenue par le procédé précédent présente un poids moléculaire apparent compris entre 25 et 27 kd. Cette hétérogénéicité peut être attribué à une modification (acylation) et/ou être due au fait que deux ATG peuvent être utilisés pour l'initiation de la traduction de F.

## EXEMPLE 5

Construction de pTGII45

Le plasmide pTGII45 contient le gène F dont la partie codant pour l'extrémité NH$_2$ terminale de la protéine a été mise en phase avec la séquence signal de la glycoprotéine rabique. Les constructions dérivent du phage MI3TGI70 décrit auparavant.

La partie 5' du gène F est mise en phase avec la séquence signal de la glycoprotéine rabique au moyen d'une délétion générée par oligonucléotide.

Les quatre premiers acides aminés de la glycoprotéine rabique sont conserves pour favoriser un bon clivage du peptide signal.

```
5'  Phe Gly Lys Phe Pro Ile   Gly Gly Lys Trp Ser Lys  3'

    TTT GGG AAA TTC CCT ATT    GGT GGC AAG TGG TCA AAA

    fin du      4 premiers             F
    signal      acides aminés   (l'ATG d'initiation est
    rage        de la glyco-            délété)
                protéine
                rabique
```

L'oligonucléotide servant à générer la boucle a pour séquence :

**5' TTTTGACCACTTGCCACCAATAGGGAATTTCCCAAA 3'**

On obtient ainsi le phage MI3TGI7I. Le signal rage (SR) de la glycoprotéine rabique fusionné avec le gène F est ensuite transféré dans le plasmide pTGI86-poly.

Pour ce faire, on clone le fragment PstI-PstI de MI3TGI7I dans pTGI86-poly ouvert au site PstI. Le plasmide résultant est nommé pTGII45. Le gène F fusionné avec le signal rage est transféré dans la vaccine comme décrit précédemment.

## EXEMPLE 6

Construction de pTGII46

Dans ce plasmide, la séquence codant pour la zone transmembranaire de la glycoprotéine rabique (TMR) est fusionnée avec la fin du gène F. Le codon TGA (stop) du gène F est compris dans la boucle de délétion générée par l'oligonucléotide, qui a pour séquence :

**5' TGCACTCAGTAATACATAGCAGTTCTTGAAGTACTC 3'**

```
Glu Tyr Phe Lys Asn Cys   Tyr Val Leu Leu Ser Ala

GAG TAC TTC AAG AAC TGC    TAT GTA TTA CTG AGT GCA

            F                         TMR
```

On obtient ainsi le phage MI3TGI72.

11

Le gène F fusionné en amont avec le signal rage et en aval avec la portion transmembranaire rage est transféré dans le plasmide pTGI86-poly.

Pour ce faire, on clone le fragment PstI-PstI de MI3TGI72 dans le plasmide pTGI86-poly, ouvert par PstI pour générer le plasmide pTGII46.

## EXEMPLE 7

Immunoprécipitation des protéines synthétisées par les virus recombinants VV.TG.FLAV.II45 et II46

En opérant comme cela a été décrit précédemment pour le plasmide pTGII47, on obtient les vecteurs vaccine hybrides correspondant aux différents plasmides préparés précédemment.

Ces vecteurs viraux seront appelés respectivement :

VV.TG.FLAV.II45

VV.TG.FLAV.II46.

Les protéines obtenues comme cela a été décrit précédemment sont testées par immunoprécipitation (figure 1). Les immunoprécipités obtenus avec le produit de VV.TG.FLAV.II45 font apparaître une protéine dont le poids moléculaire apparent est aux environs de 28 kd. La protéine mise en évidence dans le surnageant du milieu de culture est d'un poids moléculaire apparent plus élevé. Il semble que le produit du gène F soit effectivement excrété dans le surnageant de culture.

L'aspect diffus du signal autoradiographique suggérant l'existence d'une glycosylation, une manipulation de marquage a la $^{35}$S méthionine en présence de tunicamycine a été conduite. En présence de tunicamycine, on obtient une protéine de poids moléculaire apparent de 26 kd, nettement inférieur à celui constaté auparavant. Ceci indique que le produit du virus VV.TG.FLAV.II45 est glycosylé. La présence du peptide signal et de sites potentiels de N-glycosylation a entraîné une glycosylation de la protéine F. Comme la différence de poids moléculaire entre la protéine glycosylée et non glycosylée est de 2 kd, il est vraisemblable qu'un seul des deux sites de glycosylation soit utilisé.

La même expérience conduite avec le virus VV.TG.FLAV.II47 montre que le produit de ce virus n'est pas glycosylé.

Les immunoprécipités obtenus avec le produit de VV.TG.FLAV.II46 font apparaître une protéine de poids moléculaire apparent supérieur (34 kd) à ceux observés précédemment. On pouvait attendre ce résultat car le gène F est fusionné avec la partie terminale de la glycoprotéine rabique, comprenant la partie transmembranaire de cette dernière.

Il est également démontré, par marquage à la méthionine $^{35}$S en présence de tunicamycine, que le produit de VV.TG.FLAV.II46 est glycosylé. L'excrétion observée est moindre que celle constatée avec VV.TG.FLAV.II45, indiquant que la protéine est bien ancrée à la surface des cellules.

Cela est confirmé par immunofluorescence selon une technique décrite précédemment. La surface des cellules apparaît nettement fluorescente par rapport à un témoin négatif, ce qui démontre la présence de la protéine F en surface des cellules.

## EXEMPLE 8

Mise en évidence des anticorps anti-F chez des souris vaccinées avec les virus VV.TG.FLAV.II45, II46 et II47

Des souris Balb/c et C57/BI mâles de 7 semaines sont vaccinées par injection sous-cutanée de 5.10$^7$ pfu ou par scarification de la base de la queue et application de 2.10$^7$ pfu de virus VV.TG.FLAV par animal. Elles reçoivent une injection de rappel avec la même dose après 2 semaines, et subissent une prise de sang au moment du rappel ainsi que 2 et 4 semaines après le rappel. La présence d'anticorps dirigés contre des déterminants du virus LAV et du virus de la vaccine dans leurs sérums est recherchée.

Tous les animaux vaccinés donnent des sérums capables de réagir avec le virus de la vaccine dans un test ELISA.

Des techniques d'immunoprécipitation ou de "Wertern Blot" ont été utilisées. Ces méthodes permettent de mettre en évidence les anticorps capables de réagir avec les protéines du virus LAV non dénaturées ou dénaturées au SDS dans un gel d'électrophorèse et transférées sur une membrane de nitrocellulose. Dans cette expérience, les membranes de nitrocellulose employées sont obtenues par transfert des protéines contenues dans un extrait cellulaire infecté par le virus LAV selon des techniques connues. Ces membranes sont découpées en bandes et chaque bande est incubée avec le sérum des souris vaccinées (dilution au 1/20). La protéine A marquée à $^{125}$I permet de visualiser les protéines du virus LAV qui ont fixé des anticorps de souris.

Des extraits cellulaires infectés par le virus LAV et marqués à la cystéine $^{35}$S ont été utilisés pour les expériences d'immunoprécipitation.

Plusieurs sérums donnent une réaction spécifique avec une protéine de poids moléculaire autour de 27 kd, correspondant à la protéine F.

Il faut noter que les sérums de quelques souris produisent en "Western Blot" des signaux correspondant à des protéines non identifiées de la préparation de virus LAV fixée sur les membranes.

## EXEMPLE 9

Myristilation de la protéine F

On a montré que des cellules BHK2l infectées par le recombinant VV.TG.FLAV.ll47 expriment une protéine semblable à la protéine F native et qui se présente sous deux formes de poids moléculaires apparents de 25 et 27 kd en électrophorèse en gel de polyacrylamide SDS (figure 3).

La protéine F synthétisée par les cellules infectées par le virus HIV sauvage étant myristilée (Allan et al., 1985), l'existence d'une modification post-traductionnelle semblable a été recherchée sur la molécule synthétisée par le virus recombinant par marquage avec de l'acide myristique tritié.

Un tapis de cellules BHK2l ($10^6$ cellules/boîte) est infecté avec le virus recombinant (à 0,2 pfu/cellule) ; 12 heures après l'infection on ajoute 100 $\mu$Ci/ml d'acide myristique $^3$H (Amersham) dans du milieu MEM supplémenté en pyruvate de sodium 5 mM. Après 4 heures d'incubation les protéines marquées sont récoltées et immunoprécipitées avec un sérum de malades atteints de SIDA. Les protéines ayant réagi avec les anticorps sont récupérées par adsorption sur une résine de protéine A-sépharose et séparées par électrophorèse en gel de polyacrylamide SDS.

L'autoradiographie du gel montre que seule la forme de poids moléculaire apparent de 25 kd est myristilée ; la forme de 27 kd n'apparaît pas (figure 3).

L'expression de la protéine F par le virus de la vaccine recombinant permet donc d'obtenir une myristilation de la molécule semblable à celle de la molécule native synthétisée par le virus HIV.

Le myristate est probablement responsable de l'attachement de la molécule dans les membranes intracellulaires comme on l'observe pour d'autres protéines myristilées (Gould et al., 1985).

## TABLEAU 1

### Comparaison des séquences de la protéine F du HIV-1, du HIV-2 (Guyader et al., 1987) et de la protéine p60src dans leur partie N-terminale, et de la partie intracytoplasmique proche de la membrane du récepteur EGF

| | | | | | | | | | | | | | | | | | | | | | | | | |
|---|---|---|---|---|---|---|---|---|---|---|---|---|---|---|---|---|---|---|---|---|---|---|---|---|
| Myr G | G | K | W | S | K | S | S | V | V | G | W | P | T 15 | V | R | E | R | M | R R | A | E | P | A A | F HIV-1 BRU |
| M G | A | | | S | G | S | | | K K | H | | | S 10 | R | P P | R | G L | | Q E | R | L | L | | F HIV-2 ROD |
| Myr G | S | | | S | K | S | | K P | K | D | P | S 13 | Q | R R | R | S 17 | | E | P | P D | | | pp60 src |
| tm /// | R R R | H | I | V | R K | R | | T L 454 | R | | R L | L | | Q E | R | E | L | | | | | | EGF rec |

Le site de phosphorylation par la protéine kinase C de FHIV-1, pp60src et EGFrec est indiqué par la flèche. Myr = acide myristique ; tm = partie transmembranaire de la protéine EGFrec.

## EXEMPLE 10

Phosphorylation de la protéine F

La protéine F est myristilée (exemple 9) et se trouve ainsi ancrée dans la membrane au contact d'autres protéines membranaires. Il existe des analogies de séquence entre F et la protéine pp60src (Gould et al., 1985), également myristilée, et avec le récepteur EGF (Ullrich et al., 1984), dans sa partie intracytoplasmique proche de la membrane (tableau 1). Ces deux dernières protéines étant phosphorylées par la protéine kinase C sur un site proche de la membrane, il a été cherché si une telle modification existait pour F. Un site consensus de phosphorylation par la protéine kinase C existe effectivement sur la thréonine 15 de F (Woodgett et al. 1986).

Un tapis de cellules BHK2I ($10^6$ cellules/boîte) est infecté avec le recombinant VV.TG.II47 (0,1 pfu/cellule) ; 12 heures après infection on ajoute 100 $\mu$Ci/ml de $^{32}PO_4$ (Amersham) dans du milieu MEM sans phosphate. Après 4 heures d'incubation, on effectue une immunoprécipitation et une électrophorèse sur gel, comme décrit dans l'exemple 4.

La figure 3 montre que la protéine de 25 kd qui est myrtistilée est également phosphorylée. Cette protéine est résolue en deux bandes : P25-1 et P25-2. Si l'on ajoute au milieu de culture, 10 minutes avant immunoprécipitation, l'ester de phorbol 12-tetradecanoyl phorbol-13-acétate (TPA), on observe une augmentation très nette de la phosphorylation pour la P25-1. Cela est en accord avec une phosphorylation par la protéine kinase C dont l'activité est très fortement augmentée par le TPA (figure 4).

De nombreux isolats de HIV possèdent un gène F dont la thréonine 15 est remplacée par une alanine (Ratner et al., 1985). La protéine F de tels isolats est donc théoriquement non phosphorylable. Pour vérifier ce point, on a construit un recombinant exprimant une protéine F ayant une alanine en position 15.

La P25-2 peut correspondre soit à une forme de F phosphorylée sur un autre acide aminé que la Thrl5, soit à une protéine cellulaire immunoprécipitée avec F.

## EXEMPLE 11

### Construction du plasmide pTGII9I

Le plasmide pTGII9I contient le gène F provenant du plasmide pJI9-6 mais dont la thréonine en position 15 est remplacée par une alanine. Les constructions dérivent du phage MI3TGI70 décrit précédemment (exemple 2). La mutation thréonine-alanine est effectuée au moyen de l'oligonucléotide de séquence :

$$5'\ TCTTTCCCTCACTGCAGGCCATCC\ 3'$$

On obtient ainsi le phage MI3TGII5I. Le gène F-Ala est ensuite transféré dans le plasmide pTGI86-poly. Pour ce faire, on clone le fragment BamHI-SstI de MI3TGII5I dans le pTGI86-poly ouvert aux mêmes sites. Le plasmide résultant est nommé pTGII9I. Le transfert du gène F-Ala dans la vaccine est effectué comme décrit précédemment.

## EXEMPLE 12

### Immunoprécipitation des protéines synthétisées par le virus recombinant VV.TG.FLAV.II5I

En opérant comme cela a été décrit précédemment pour le plasmide pTGII47, on obtient le vecteur viral VV.TG.FLAV.II9I.

Les protéines obtenues sont examinées par immunoprécipitation après marquage à la $^{35}S$ méthionine comme décrit précédemment. Les protéines obtenues et visualisées sur gel SDS sont identiques à celles induites par le recombinant VV.TG.FLAV.II47 (non montré).

Si l'on effectue un marquage au phosphate $^{32}PO_4$ comme décrit plus haut, on ne visualise plus la forme P25-1 induite par le recombinant VV.TG.FLAV.II47. De plus, l'addition de TPA dans le milieu ne modifie pas la phosphorylation de la forme P25-2 (figure 4).

Ces résultats indiquent que le site de phosphorylation par la protéine kinase C est la thréonine 15. Le fait que la protéine F de certains isolats ne soit pas phosphorylable par la PKC peut impliquer une activité biologique différente pour ces isolats. En effet, la phosphorylation par la PKC constitue généralement une régulation de l'activité de ses substrats (voir revue par Nishizuka, 1986).

## EXEMPLE 13

### Construction du plasmide pTGII65

Il a été montré que l'immunogénicité de la protéine F était accrue si celle-ci était couplée à une zone hydrophobe en C-terminal (recombinant VV.TG.FLAV.II46). Cependant, il est apparu que la conformation de la protéine n'était pas correcte car celle-ci est alors très peu reconnue par des anticorps d'invidus séropositifs. Il a donc été entrepris la construction d'un virus recombinant exprimant la protéine F couplée à un peptide hydrophobe en N-terminal. Ce peptide jouerait ainsi le rôle de l'acide myristique, tout en assurant un ancrage plus solide de la protéine dans la membrane cellulaire. Ce recombinant a été construit à partir du recombinant VV.TG.FLAV.II45 décrit précédemment. Le présent exemple concerne donc l'utilisation d'un vecteur viral exprimant une protéine F plus immunogène que la protéine F non modifiée, et ayant une conformation similaire à la protéine native.

Le plasmide pTGII65 contient donc le gène F dont la partie codant pour l'extrémité $NH_2$ terminale de la protéine a eté mise en phase avec la séquence signal de la glycoprotéine rabique, cette séquence signal était mutée afin de supprimer le clivage in vivo entre le peptide signal hydrophobe et la protéine F. Les constructions dérivent du phage MI3TGI7I décrit précédemment (exemple 5).

Le site de clivage est situé entre la glycine terminale du peptide signal et la lysine N-terminale de la glycoprotéine rabique. Afin de supprimer le clivage, la glycine située en -1 du site de clivage a été mutée en tryptophane, la phénylalanine en -2 a été mutée en isoleucine et la proline en -5 en leucine. Ces mutations ont été réalisées au moyen de l'oligonucléotide de séquence :

$$5' \quad AGGGAATTTCCATAAACACAATAGAAAAACCAG \quad 3'$$

On obtient ainsi le phage MI3TGII07. La séquence signal mutée de la glycoprotéine rabique fusionnée avec le gène F est ensuite transférée dans le plasmide pTGI86-poly.

Pour ce faire, on clone le fragment PstI-PstI de MI3TGII07 dans le pTGI86-poly ouvert au site PstI. Le plasmide résultant est nommé pTGII65. Le gène F fusionné avec la séquence signal de la rage mutée est transféré dans la vaccine comme décrit précédemment.

## EXEMPLE 14

Immunoprécipitation des protéines synthétisées par le virus recombinant VV.TG.FLAV.II65

En opérant comme cela a été décrit pour le plasmide pTGII47, on obtient le virus de la vaccine recombinant correspondant au plasmide pTGII65 et dénommé VV.TG.FLAV.II65. Les protéines obtenues comme cela a été décrit précédemment sont testées par immunoprécipitation (figure 5).

Les immunoprécipités obtenus avec le produit de VV.TG.FLAV.II65 font apparaître une protéine dont le poids moléculaire apparent est de $^{26\,000}$daltons. La protéine est absente dans les surnageants de culture. Une manipulation de marquage à la $^{35}S$ méthionine en présence de tunicamycine indique que la protéine obtenue n'est pas glycosylée.

## EXEMPLE 15

Mise en évidence des anticorps anti-F chez des souris vaccinées avec les virus VV.TG.FLAV.II65

Des souris Balb/C femelles de 7 semaines sont vaccinées par scarification à la base de la queue comme décrit dans l'exemple 8. La détection des anticorps s'effectue par technique de "Western blot". La protéine F produite dans E. coli est transférée sur nitrocellulose, des bandelettes sont découpées et incubées avec les sérums des animaux vaccinés (figure 6a). La réaction observée est environ 3 à 5 fois plus forte pour le recombinant VV.TG.FLAV.II65 que pour le recombinant VV.TG.FLAV.II47, et équivalente à celle du recombinant VV.TG.FLAV.II96 (figure 6b). Cependant, l'utilisation du recombinant VV.TG.FLAV.II65 est préférable car la protéine synthétisée est parfaitement reconnue par des anticorps d'individus séropositifs, donc sa conformation est correcte. Le recombinant VV.TG.FLAV.II65 semble être le meilleur candidat pour un vaccin contre la protéine F.

Dépôt des souches représentatives de l'invention :

Le phage MI3mp70I et le plasmide pTGII34 sont décrits dans la demande de brevet n° 86 05043 du 8 avril 1986.

Le plasmide pJI9-6 a été déposé le 16 novembre 1984 à la Collection Nationale de Cultures de Microorganismes de l'Institut Pasteur sous le n° 366-I et est décrit dans le brevet anglais n° 84 29099.

La souche suivante a été déposée le 6 juin 1986 à la Collection Nationale de Cultures de Microorganismes de l'Institut Pasteur : - E. coli transformé pTGII47 sous le n° I-56I.

## REFERENCES

1. Allan J.S., Coligan J.E., Lee T.H., McLane M.F., Kanki P.J., Groopman J.E. et Essex M. (1985) Science 230, 810-813.

2. Auffray C. (1986) C.R. Acad. Sc. Paris 302 série III, N° 8, 287-292.

3. Arya K.S. et Gallo R.C. (1986) Proc. Natl. Acad. Sci. USA 83, 2209-2213.

4. Drillien R., Spehner D. (1983) Virology 131, 385-393.

5. Gould K.L., Woodgett J.R., Cooper J.A., Buss J.E., Shalloway D.F. et Hunter T. Cell 42, 849-857 (1985).

6. Guyader M., Guerneau M., Sonigo P., Clavel F., Montagnier L. et Alizon M. Nature 328, 662-669 (1987).5.

7. Kieny M.P., Lathe R. et Lecocq J.P., 1983, Gene 26 : 91-99.

8. Kieny M.P., Lathe R., Drillien R., Spehner D., Skory S., Schmitt D., Wiktor T., Koprowski H. et Lecocq. J.P. (1984) Nature 312: 163-166.

9. Lathe R., Hirth P., Dewilde M., Harford N et Lecocq J.P., 1980, Nature 284 : 473-474.

10. Lathe P., Kieny M.P., Schmitt D., Curtis P., Lecocq J.P. (1984) J. Mol. Appl. Genet., vol. 2, 331-342.

11. Lathe P. Kieny M.P., Skory S. et Lecocq J.P. (1984) DNA, vol. 3, 173-182.

12. Lusky M., Botchan M. (1981) Nature 293? 79-81.

13. Mackett M., Smith G.L., et Moss B., (1982) Proc. Natl. Acad. Sci. USA 79 : 7415-7419.

14. Maniatis T., Fritsch E.F., Sambrook J. (1982) Cold Spring Harbor Lab. N.Y.

15. Muesing M.A., Smith D.H., Cabradilla C.D., Benton C.V., Lasky L.A. et Capon D.J. (1985) Nature 313 : 450-458.

16. Messing et Vieras, Gene 19, (1982) 269-276.

17. Nishizuka Y., (1986) Science 233, 305-312.

18. Panicali D. et Paoletti E. (1982) Proc. Natl. Acad. Sci. USA 79 : 4927-4931.

19. Panicali D., Davis S.W., Weinberg R.L., Paoletti E. (1983) Proc. Natl. Acad. Sci. USA 80, 5364-5368.

20. Ratner L., Haseltine W., Patarca R., Livak K.J., Starcich B., Josephs S.F., Doran E.R., Rafalski J.A., Whitehorn E.A., Baumeister K., Ivanoff L., Petterway Jr. S.R., Pearson M.L., Lautenberger J.A., Papas T.S., Ghrayeb J., Chang N.T., Gallo R.C. et Wong-Staal F. (1985) 313 : 277-284.

21. Patner L., Stracich B., Joseph S.F., Hahn B.H., Reddy E.P., Livak K.J., Petterway S.R., Pearson M.L., Haseltine W.A., Arya K.S. et Wong-Staal F. (1985) Nucleic Acids Research, 13, 8219-8229.

22. Sanchez-Pescador et al. (1985) Science, 227 : 484-492.

23. Smith G.L., Mackett M. Moss Y. (1983) Nature 302 : 490-495.

24. Smith G.L., Murphy B.R., Moss B. (1983) Proc. Natl. Acad. Sci. USA 80, 7155-7159.

25. Ullrich et al. Nature 309 : 418-425 (1984).

26. Venkatesan S., Baroudy B.M., Moss B. (1981) Cell 125 : 805-813.

27. Wain-Hobson S., Sonigo P., Danos O., Cole S. et Alizon M. (1985) Cell 40 : 9-17.

28. Weir J.P., Moss B. (1983) J. Virol. 46 : 530-537.

29. Woodgett J.P., Gould K.L. et Hunter T. Eur. J. Biochem. 161 : 177-184 (1986).

30. Zoller M.J. et Smith M. (1983) Methods in Enzymology (Wu, Grossman, Moldave, eds.) 100 : 468-500.

**Revendications**

**Revendications pour les Etats contractants suivants : BE, CH, DE, FR, GB, IT, LI, LU, NL, SE**

1. Vecteur viral caractérisé en ce qu'il comporte au moins :
   . une partie du génome d'un virus,
   . un gène codant pour la protéine F du virus responsable du SIDA,
   . ainsi que les éléments assurant l'expression de cette protéine dans des cellules, et une séquence signal et/ou une séquence transmembranaire provenant d'un virus hétérologue.

2. Vecteur viral selon la revendication 1, caractérisé en ce que la séquence signal et/ou la séquence transmembranaire provenant d'un virus hétérologue provient du virus de la rage.

3. Vecteur viral selon l'une des revendications 1 ou 2, caractérisé en ce que le gène codant est le gène F total comportant depuis l'extrémité 5' :
   . la séquence signal du gène G du virus de la rage,
   . le gène F total,
   . la zône transmembranaire (TM) du gène G.

4. Vecteur viral selon les revendications 1 à 3, caractérisé en ce que la partie du génome d'un virus est une partie du génome d'un poxvirus.

**5.** Vecteur viral selon la revendication 4, caractérisé en ce que le poxvirus est la vaccine.

**6.** Vecteur viral selon l'une des revendications 1 à 5, caractérisé en ce que la séquence d'ADN codant pour la protéine F est sous la dépendance d'un promoteur d'un gène du poxvirus.

**7.** Vecteur viral selon la revendication 6 , caractérisé en ce que le promoteur est un promoteur du gène de la vaccine.

**8.** Vecteur viral selon la revendication 7 , caractérisé en ce que la séquence d'ADN codant pour la protéine F est sous le contrôle du promoteur du gène de la protéine 7,5K de la vaccine.

**9.** Vecteur viral selon l'une des revendications 7 et 8, caractérisé en ce que la séquence codant pour la protéine F est clonée dans le gène TK de la vaccine.

**10.** ADN recombinant correspondant à un vecteur viral selon l'une des revendications 1 à 9.

**11.** Culture de cellules de mammifères infectées par un vecteur viral selon l'une des revendications 1 à 9 ou comportant un ADN selon la revendication 10.

**12.** Procédé de préparation de la protéine F du virus responsable du SIDA, caractérisé en ce qu'on cultive des cellules selon la revendication 11 et qu'on récupère la protéine F produite.

**13.** Protéine F de virus responsable du SIDA obtenue par mise en oeuvre du procédé selon la revendication 12 et caractérisée ence qu'elle comporte,en outre, une partie de glycoprotéine de la rage.

**14.** Protéine F de virus responsable du SIDA obtenue par mise en oeuvre du procédé selon la revendication 12 et caractérisée en ce qu'elle comporte, en outre, un peptide hydrophobe en position N-terminale.

**15.** Protéine selon la revendication 14, caractérisée en ce qu'elle comporte, en outre, un peptide hydrophobe en position C-terminale.

**16.** Vaccin caractérisé en ce qu'il est constitué par un vecteur viral selon l'une des revendications 1 à 10 et/ou par la protéine selon l'une des revendications 14 ou 15.

**17.** Anticorps dressés contre la protéine F du virus responsable du SIDA, caractérisés en ce qu'on inocule un organisme vivant avec un vecteur viral selon l'une des revendications 1 à 10 ou avec les protéines obtenues selon l'une des revendications 14 ou 15 et en ce qu'on récupère les anticorps formés après un temps déterminé.

**18.** Procédé de diagnostic in vitro du SIDA chez un patient à l'aide des anticorps de la revendication 17 dans un prélèvement biologique dudit patient.

**Revendications pour les Etats contractants suivants : AT, ES, GR**

**1.** Procédé de préparation d'un vecteur viral caractérisé en ce qu'on assemble au moins:
- une partie du génome d'un virus,
- un gène codant pour la protéine F du virus responsable du SIDA,
- ainsi que les éléments assurant l'expression de cette protéine dans des cellules, et une séquence signal et/ou une séquence transmembranaire provenant d'un virus hétérologue.

**2.** Procédé selon la revendication 1, caractérisé en ce que la séquence signal et/ou la séquence transmembranaire provenant d'un virus hétérologue provient du virus de la rage.

**3.** Procédé selon l'une des revendications 1 ou 2, caractérisé en ce que le gène codant est le gène F total comportant depuis l'extrémité 5' :
- la séquence signal du gène G du virus de la rage,
- le gène F total,

- la zone transmembranaire (TM) du gène G.

4. Procédé selon les revendications 1 à 3, caractérisé en ce que la partie du génome d'un virus est une partie du génome d'un poxvirus.

5. Procédé selon la revendication 4, caractérisé en ce que le poxvirus est la vaccine.

6. Procédé selon l'une des revendications 1 à 5, caractérisé en ce que la séquence d'ADN codant pour la protéine F est sous la dépendance d'un promoteur d'un gène du poxvirus.

7. Procédé selon la revendication 6, caractérisé en ce que le promoteur est un promoteur du gène de la vaccine.

8. Procédé selon la revendication 7, caractérisé en ce que la séquence d'ADN codant pour la protéine F est sous le contrôle du promoteur du gène de la protéine 7,5K de la vaccine.

9. Procédé selon l'une des revendications 7 et 8, caractérisé en ce que la séquence codant pour la protéine F est clonée dans le gène TK de la vaccine.

10. Procédé de préparation de la protéine F du virus responsable du SIDA, caractérisé en ce qu'on cultive des cellules de mammifères infectées par un vecteur viral obtenu par un procédé selon l'une des revendications 1 à 9 ou comportant un ADN correspondant à ce vecteur viral et qu'on récupère la protéine F produite.

11. Procédé selon la revendication 10 caractérisé en ce que la protéine F comporte, en outre, une partie de glycoprotéine de la rage.

12. Procédé selon la revendication 10 caractérisé en ce que la protéine F comporte, en outre, un peptide hydrophobe en position N-terminale.

13. Procédé selon la revendication 12 caractérisé en ce que la protéine F comporte, en outre, un peptide hydrophobe en position C-terminale.

14. Utilisation d'un vecteur viral obtenu par le procédé selon l'une des revendications 1 à 9 et/ou de la protéine obtenue par le procédé selon l'une des revendications 11 à 13 pour la préparation d'un vaccin.

15. Procédé de préparation d'un anticorps dressé contre la protéine F du virus responsable du SIDA, caractérisé en ce qu'on inocule un organisme vivant avec un vecteur viral obtenu par le procédé selon l'une des revendications 1 à 9 ou avec les protéines obtenues par le procédé selon l'une des revendications 11 à 13 et en ce qu'on récupère les anticorps formés après un temps déterminé.

16. Procédé de diagnostic in vitro du SIDA chez un patient à l'aide des anticorps de la revendication 15 dans un prélèvement biologique dudit patient.

**Claims**
**Claims for the following Contracting States : BE, CH, DE, FR, GB, IT, LI, LU, NL, SE**

1. Viral vector characterized in that it contains at least:
   . a portion of the genome of a virus,
   . a gene coding for the F protein of the virus responsible for AIDS,
   . and also the elements which provide for the expression of this protein in cells and a signal sequence and/or a transmembrane sequence originating from a heterologous virus.

2. Viral vector according to Claim 1, characterized in that the signal sequence and/or the transmembrane sequence originating from a heterologous virus originate(s) from the rabies virus.

3. Viral vector according to one of Claims 1 and 2, characterized in that the coding gene is the whole F gene containing, from the 5' end:

19

. the signal sequence of the G gene of the rabies virus,
. the whole F gene,
. the transmembrane (TM) region of the G gene.

4. Viral vector according to Claims 1 to 3, characterized in that the portion of the genome of a virus is a portion of a genome of a poxvirus.

5. Viral vector according to Claim 4, characterized in that the poxvirus is vaccinia.

6. Viral vector according to one of Claims 1 to 5, characterized in that the DNA sequence coding for the P protein is under the control of a promoter of a poxvirus gene.

7. Viral vector according to Claim 6, characterized in that the promoter is a promoter of the vaccinia gene.

8. Viral vector according to Claim 7, characterized in that the DNA sequence coding for the F protein is under the control of the promoter of the gene for the 7.5K protein of vaccinia.

9. Viral vector according to either of Claims 7 and 8, characterized in that the sequence coding for the F protein is cloned into the TK gene of vaccinia.

10. Recombinant DNA corresponding to a viral vector according to one of Claims 1 to 9.

11. Culture of mammalian cells infected with a viral vector according to one of Claims 1 to 9 or containing a DNA according to Claim 10.

12. Method for preparing the F protein of the virus responsible for AIDS, characterized in that cells are cultured according to Claim 11 and in that the F protein produced is recovered.

13. F protein of virus responsible for AIDS, obtained by carrying out the method according to Claim 12 and characterized in that it contains, in addition, a portion of rabies glycoprotein.

14. F protein of virus responsible for AIDS, obtained by carrying out the method according to Claim 12 and characterized in that it contains, in addition, a hydrophobic peptide at the N-terminal position.

15. Protein according to Claim 14, characterized in that it contains, in addition, a hydrophobic peptide at the C-terminal position.

16. Vaccine, characterized in that it consists of a viral vector according to one of Claims 1 to 10 and/or the protein according to one of Claims 14 and 15.

17. Antibodies raised against the F protein of the virus responsible for AIDS, characterized in that a living organism is inoculated with a viral vector according to one of Claims 1 to 10 or with the proteins obtained according to one of Claims 14 and 15, and in that the antibodies formed are recovered after a specified time.

18. Method for the in vitro diagnosis of AIDS in a patient, using the antibodies of Claim 17 in a biological sample taken from the said patient.

**Claims for the following Contracting States : AT, ES, GR**

1. Method for preparing a viral vector, characterized in that at least:
   . a portion of the genome of a virus,
   . a gene coding for the F protein of the virus responsible for AIDS,
   . and also the elements which provide for the expression of this protein in cells, and a signal sequence and/or a transmembrane sequence originating from a heterologous virus,
   are assembled.

2. Method according to Claim 1, characterized in that the signal sequence and/or the transmembrane sequence originating from a heterologous virus originate(s) from the rabies virus.

3. Method according to either of Claims 1 and 2, characterized in that the coding gene is the whole F gene containing, from the 5' end:
   . the signal sequence of the G gene of the rabies virus,
   . the whole F gene,
   . the transmembrane (TM) region of the G gene.

4. Method according to Claims 1 to 3, characterized in that the portion of the genome of a virus is a portion of the genome of a poxvirus.

5. Method according to Claim 4, characterized in that the poxvirus is vaccinia.

6. Method according to one of Claims 1 to 5, characterized in that the DNA sequence coding for the F protein is under the control of a promoter of a poxvirus gene.

7. Method according to Claim 6, characterized in that the promoter is a promoter of the vaccinia gene.

8. Method according to Claim 7, characterized in that the DNA sequence coding for the F protein is under the control of the promoter of the gene for the 7.5K protein of vaccinia.

9. Method according to either of Claims 7 and 8, characterized in that the sequence coding for the F protein is cloned into the TK gene of vaccinia.

10. Method for preparing the F protein of the virus responsible for AIDS, characterized in that mammalian cells infected with a viral vector obtained by a method according to one of Claims 1 to 9, or containing a DNA corresponding to this viral vector, are cultured, and in that the F protein produced is recovered.

11. Method according to Claim 10, characterized in that the F protein contains, in addition, a portion of rabies glycoprotein.

12. Method according to Claim 10, characterized in that the F protein contains, in addition, a hydrophobic peptide at the N-terminal position.

13. Method according to Claim 12, characterized in that the F protein contains, in addition, a hydrophobic peptide at the C-terminal position.

14. Use of a viral vector obtained by the method according to one of Claims 1 to 9 and/or of the protein obtained by the method according to one of Claims 11 to 13 for the preparation of a vaccine.

15. Method for preparing an antibody raised against the F protein of the virus responsible for AIDS, characterized in that a living organism is inoculated with a viral vector obtained by the method according to one of Claims 1 to 9 or with the proteins obtained by the method according to one of Claims 11 to 13, and in that the antibodies formed are recovered after a specified time.

16. Method for the in vitro diagnosis of AIDS in a patient, using the antibodies of Claim 15 in a biological sample taken from the said patient.

**Patentansprüche**
**Patentansprüche für folgende Vertragsstaaten : BE, CH, DE, FR, GB, IT, LI, LU, NL, SE**

1. Viraler Vektor, dadurch charakterisiert, daß er mindestens umfaßt:
   . einen Teil des Genoms eines Virus
   . ein für das Protein F des für AIDS verantwortlichen Virus codierendes Gen
   . ferner die die Expression dieses Proteins in Zellen sicherstellenden Elemente, und eine von einem heterologen Virus stammende Signalsequenz und/oder transmembranale Sequenz.

**2.** Viraler Vektor nach Anspruch 1, dadurch charakterisiert, daß die von einem heterologen Virus stammende Signalsequenz oder transmembranale Sequenz vom Tollwutvirus stammt.

**3.** Viraler Vektor nach einem der Ansprüche 1 oder 2, dadurch charakterisiert, daß es sich bei dem codierenden Gen um das gesamte Gen F handelt, umfassend vom 5' -Ende an:
  . die Signalsequenz des Gens G des Tollwutvirus,
  . das gesamte Gen F
  . die transmembranale Zone (TM) des Gens G.

**4.** Viraler Vektor nach den Ansprüchen 1 bis 3, dadurch charakterisiert, daß es sich bei dem Teil des Genoms eines Virus um ein Teil des Genoms des Poxvirus (Pockenvirus) handelt.

**5.** Viraler Vektor nach Anspruch 4, dadurch charakterisiert, daß es sich bei dem Poxvirus um das Vaccinevirus handelt.

**6.** Viraler Vektor nach einem der Ansprüche 1 bis 5, dadurch charakterisiert, daß die für das Protein F codierende DNA-Sequenz abhängig ist von einem Promoter eines Gens des Poxvirus.

**7.** Viraler Vektor nach Anspruch 6, dadurch charakterisiert, daß es sich bei dem Promoter um einen Promoter des Vaccinevirus-Gens handelt.

**8.** Viraler Vektor nach Anspruch 7, dadurch charakterisiert, daß die für das Protein F codierende DNA-Sequenz unter der Kontrolle des Promoters des Gens für das Protein 7,5K des Vaccinevirus steht.

**9.** Viraler Vektor nach einem der Ansprüche 7 und 8, dadurch gekennzeichnet, daß die für das Protein f codierende Sequenz im Gen TK des Vaccinevirus kloniert ist.

**10.** Rekombinante DNA, entsprechend einem viralen Vektor nach einem der Ansprüche 1 bis 9.

**11.** Kultur von Säugerzellen, die infiziert sind mit einem viralen Vektor nach einem der Ansprüche 1 bis 9 oder die eine DNA nach Anspruch 10 umfassen.

**12.** Verfahren zur Herstellung des Proteins F des für AIDS verantwortlichen Virus, dadurch charakterisiert, daß man Zellen nach Anspruch 11 kultiviert und das produzierte Protein F gewinnt.

**13.** Protein F des für AIDS verantwortlichen Virus, erhalten dadurch, daß man das Verfahren nach Anspruch 12 durchführt und dadurch charakterisiert, daß es zusatzlich einen Teil des Glykoproteins des Tollwutvirus trägt.

**14.** Protein F des für AIDS verantwortlichen Virus, erhalten dadurch, daß man das Verfahren nach Anspruch 12 durchführt und dadurch charakterisiert, daß es zusätzlich in der N-terminalen Position ein hydrophobes Peptid trägt.

**15.** Protein nach Anspruch 14, dadurch charakterisiert, daß es zusätzlich in der C-terminalen Position ein hydrophobes Peptid trägt.

**16.** Impfstoff, dadurch charakterisiert, daß er aus einem viralen Vektor nach einem der Ansprüche 1 bis 10 und/oder aus dem Protein nach einem der Ansprüche 14 oder 15 besteht.

**17.** Gegen das Protein F des für AIDS verantwortlichen Virus gerichtete Antikörper, dadurch charakterisiert, daß man einen lebenden Organismus mit einem viralen Vektor nach einem der Ansprüche 1 bis 10 oder mit den Proteinen nach einem der Ansprüche 14 oder 15 impft und nach einer bestimmten Zeit die gebildeten Antikörper gewinnt.

**18.** Verfahren zur in-vitro-Diagnose von AIDS bei einem Patienten mit Hilfe von Antikörpern nach Anspruch 17 in einer biologischen Ausscheidung des Patienten.

EP 0 253 693 B1

**Patentansprüche für folgende Vertragsstaaten : AT, ES, GR**

1. Verfahren zur Herstellung eines viralen Vektors, dadurch charakterisiert, daß man mindestens zusammenfügt:
   - einen Teil eines Genoms eines Virus,
   - ein für das Protein F des für AIDS verantwortlichen Virus codierendes Gen
   - ferner die die Expression dieses Proteins in Zellen sicherstellenden Elemente, und eine von einem heterologen Virus stammende Signalsequenz und/oder transmembranale Sequenz.

2. Verfahren nach Anspruch 1, dadurch charakterisiert, daß die von einem heterologen Virus stammende Signalsequenz und/oder transmembranale Sequenz vom Tollwutvirus stammt.

3. Verfahren nach einem der Ansprüche 1 oder 2, dadurch charakterisiert, daß es sich bei dem codierenden Gen um das gesamte Gen F handelt, umfassend vom 5' -Ende an:
   - die Siganalsequenz des Gens G des Tollwutvirus,
   - das gesamte Gen F
   - die transmembranale Zone (TM) des Gens G.

4. Verfahren nach einem der Ansprüche 1 bis 3, dadurch charakterisiert, daß es sich bei dem Teil des Genoms eines Virus um ein Teil des Genoms eines Poxvirus (Pockenvirus) handelt.

5. Verfahren nach Anspruch 4, dadurch charakterisiert, daß es sich bei dem Poxvirus um das Vaccinevirus handelt.

6. Verfahren nach einem der Ansprüche 1 bis 5, dadurch charakterisiert, daß die für das Protein F codierende DNA-Sequenz abhängig ist von einem Promoter eines Gens des Poxvirus.

7. Verfahren nach Anspruch 6, dadurch charakterisiert, daß es sich bei dem Promoter um einen Promoter des Gens des Vaccinevirus handelt.

8. Verfahren nach Anspruch 7, dadurch charakterisiert, daß die für das Protein F codierende DNA-Sequenz unter der Kontrolle eines Promoters des Gens des Proteins 7,5K des Vaccinevirus steht.

9. Verfahren nach einem der Ansprüche 7 und 8, dadurch charakterisiert, dass die für das Protein F codierende Sequenz im Gen TK des Vaccinevirus kloniert ist.

10. Verfahren zur Herstellung des Proteins F des für AIDS verantwortlichen Virus, dadurch charakterisiert, daß man Säugerzellen kultiviert, die infiziert sind mit einem viralen Vektor, erhalten nach einem der Ansprüche 1 bis 9 oder umfasend eine diesem viralen Vektor entsprechende DNA, und das produzierte Protein F gewinnt.

11. Verfahren nach Anspruch 10, dadurch charakterisiert, daß das Protein F zusätzlich einen Teil des Glykoproteins des Tollwutvirus umfaßt.

12. Verfahren nach Anspruch 10, dadurch charakterisiert, daß das Protein F zusätzlich in der N-terminalen Position ein hydrophobes Peptid trägt.

13. Verfahren nach Anspruch 12, dadurch charakterisiert, daß das Protein F zusätzlich in der C-terminalen Position ein hydrophobes Peptid trägt.

14. Verwendung eines nach dem Verfahren nach einem der Ansprüche 1 bis 9 erhaltenen viralen Vektors und/oder des nach dem Verfahren nach einem der Ansprüche 11 bis 13 erhaltenen Proteins für die Herstellung eines Impfstoffs.

15. Verfahren zur Herstellung eines gegen das Protein F des für AIDS verantwortlichen Virus gerichteten Antikörpers, dadurch charakterisiert, daß man einen lebenden Organismus mit einem nach einem der Ansprüche 1 bis 9 erhaltenen viralen Vektor oder mit den nach dem Verfahren eines der Ansprüche 11 bis 13 erhaltenen Proteinen impft und nach einer bestimmten Zeit die gebildeten Antikörper gewinnt.

**16.** Verfahren zur in-Vitro-Diagnose von AIDS bei einem Patienten mit Hilfe der Antikörper nach Anspruch 15 in einer biologischen Ausscheidung des Patienten.

FIG.1

FIG 2

FIG_3

FIG_4

FIG_5

FIG_6a

FIG. 6b